Europäisches Patentamt

European Patent Office

Office européen des brevets

(11) Publication number: 0 638 306 A1

# EUROPEAN PATENT APPLICATION

(21) Application number: 94110616.3

(51) Int. Cl.6: A61K 7/06

(22) Date of filing: 07.07.94

(30) Priority: 09.07.93 US 89362
14.06.94 US 259582

(43) Date of publication of application:
15.02.95 Bulletin 95/07

(84) Designated Contracting States:
AT BE DE DK ES FR GB IT NL

(71) Applicant: National Starch and Chemical
Investment Holding Corporation
501 Silverside Road
Wilmington, Delaware 19809 (US)

(72) Inventor: Guth, Jacob J.
No 2 124A Lonely Cottage Road
Upper Black Eddy,
Pennsylvania 18972 (US)
Inventor: Russo, Julie
50 Eiseman Avenue
Piscataway,
New Jersey 08854 (US)
Inventor: Mudge, Paul R.
23 Mulford Lane
Belle Mead,
New Jersey 08502 (US)
Inventor: Kay, Troy
3304 Covenanter Drive
Bloomington,
Indiana 47401 (US)
Inventor: Gallaway, George L.
RD Box 162, W. Portal
Asbury,
New Jersey 08802 (US)
Inventor: Martino, Gary T.
52 Bradford Lane
Plainsboro,
New Jersey 08536 (US)
Inventor: Wanigatunga Sirisoma
694 Foothill Road
Bridgewater
New Jersey 08807 (US)

(74) Representative: Hagemann, Heinrich, Dr. rer.
nat., Dipl.-Chem. et al
Patentanwälte
Hagemann & Kehl
Postfach 86 03 29
D-81630 München (DE)

(54) Low molecular weight hair fixative polymers in low volatile organic compounds (VOC) formulation.

(57) This invention relates to the use of low molecular weight polymers in a high solids hair spray composition containing levels of volatile organic compounds 80% or lower by weight. The hair fixative polymers suitable for use in these hair sprays are based on acrylic and vinyl-ester resins that are water soluble with neutralization. The polymers have intrinsic viscosities ranging from 0.100 to 0.300 and are present in amounts ranging from 7 weight percent to 20 weight percent, based on the total weight of the hair spray composition. The use of a lower molecular weight polymer reduces the viscosity, enhances the spray characteristics, and promotes the hydrolytic stability of the system. The high solids content compensates for any lower fixative properties generally associated with lower molecular weight hair care polymers.

Rank Xerox (UK) Business Services
(3.10/3.09/3.3.4)

This invention relates to the use of low molecular weight, hydrolytically stable polymers in a high solids hair spray formulation containing levels of volatile organic compounds 80% or lower by weight.

Most hair spray formulations contain a film-forming polymer in an alcohol or a mixture of alcohol and water delivery system. In the case of aerosol delivery, the delivery system will also contain a propellant, which is typically a volatile hydrocarbon. Such aerosol systems are discussed in, for instance, United States Patent 3,810,977, Levine et al., wherein hair fixing terpolymer compositions of vinyl acetate, crotonic acid and at least one vinyl ester of an alpha-branched saturated aliphatic monocarboxylic acid having from 5 to 10 carbon atoms in the carboxyl acid moiety are disclosed. The polymers may contain from 7% to 89% of vinyl ester of an alpha-branched saturated aliphatic monocarboxylic acid having from 5 to 10 carbon atoms in the carboxyl acid moiety are disclosed. The polymers may contain from 7% to 89% of vinyl acetate and from about 5% to 80% of the selected vinyl ester.

United States Patent 4,567,035, Waxman et al., discloses a low molecular weight hair spray resin containing a vinyl monomer having an electron withdrawing group as described therein and consisting essentially of alternating units of vinyl ester and an alkyl half ester maleate derived from alternating vinyl ester/maleic anhydride copolymer. It is stated that using resins having alternating monomer structure and containing an approximate 50/50 mole ratio of vinyl ester/maleic anhydride half ester units is important in providing the soap solution solubility of the hair spray resin. Accordingly, it is essential that the "Waxman" resins be derived from a preformed vinyl ester/maleic anhydride alternating copolymer under specific reaction conditions for subsequent esterification of the anhydride group.

United States Patent 3,927,199, Micchelli et al., discloses hair fixative compositions containing as the film forming resinous binder an interpolymer derived from (1) N-alkyl acrylamides or methacrylamides, (2) acidic film forming comonomers, and (3) at least one polymerizable comonomer. The interpolymer is said to comprise 30-60% of the N-substituted acrylamide or methacrylamide.

In order to be effective in a hair fixative composition, such as aerosol sprays in which alcohols and hydrocarbon delivery systems are used, the film forming polymeric binders utilized therein, as well as the films formed therefrom, must meet certain performance criteria. Initially, the polymers must be soluble or dispersable in the particular solvent system in which they are used, yet the films formed therefrom must be readily removable by the use of water and/or soap or shampoo. Additionally, the films must exhibit good strength and flexibility simultaneously. In some states environmental regulations controlling the emission of volatile organic compounds (VOCs) into the atmosphere require that these alcohol and hydrocarbon delivery systems contain 80% or less volatile organic compounds, and soon may require emissions to be 55% or less. Due to these regulations, it is foreseen that water will become a greater component in hair spray formulations. However, the use of water as a solvent for many hair fixative polymers in current use reduces the performance properties of the polymers, particularly in the area of spray characteristics, hydrolytic stability, initial curl droop, i.e. stiffness, and humidity resistance.

United States Patent 5,206,009, Watling et al., discloses non-aerosol, low VOC, pump hair spray compositions which consist essentially of a hair fixative polymer, and a mixture of alcohol, water and dimethoxymethane as cosolvents therefor. Watling does not address the problems of hydrolytic stability in hair fixatives which contain large amounts of water, nor is there any teaching contained therein regarding sprayability problems of aqueous-based hair fixative aerosol sprays.

This invention presents hair spray compositions that contain 80% or less by total weight of volatile organic compounds (VOC) and that exhibit good spray characteristics, good curl retention with minimal initial curl droop, i.e., good humidity resistance and stiffness, good removability by water and/or soap or shampoo, and hydrolytic stability. These properties are obtained by the use of a relatively high solids content of hydrophobic, low molecular weight, hydrolytically stable, film-forming hair fixative polymers which are based on acrylic or vinyl-ester resins that are soluble in water upon neutralization. As used herein, water soluble is meant to include both water soluble in its usual meaning as well as water dispersible wherein the resulting films are sufficiently hydrated and softened by contact with water so as to be easily removed from the hair by the application of water and either soap or shampoo. The hair fixative polymers utilized in the hair spray compositions will have a preferred number average molecular weight of from 10,000 to 30,000, as indicated by an intrinsic viscosity of from 0.100 to 0.300.

The general classes of hair fixative resins suitable for use in the hair sprays of the present invention are those described in United States Patents 3,810,977 (vinyl ester) and 3,927,199 (acrylic), hereby incorporated by reference. However, the hair fixing compositions disclosed therein contain significant amounts, i.e., greater than 80%, of volatile organic compounds (VOC) in the form of organic solvent. As used herein, a volatile organic compound is an organic compound containing at least one, but not more than 10, carbon atoms, except for those organic compounds having a vapor pressure of less than 0.1 mm Hg at 20°C. Suitable examples of volatile organic compounds are ethanol, isopropanol, acetone, dimethyl ether, dimethoxymethane and methyl ethyl ketone. Neither system anticipates problems associated with the presence in the hair fixing composition of significant amounts of water. The resins disclosed therein are relatively high molecular weight resins compared to the polymers of the present invention Accordingly, the use of those polymers at the relatively high concentrations required by the hair fixative compositions of the present invention lead to poor spray characteristics.

As one skilled in the art will appreciate, molecular weight determinations are typically measured relative to some standard. As one skilled in the art will also appreciate, intrinsic viscosity is one property of polymers which is directly proportional to the molecular weight, number average or weight average. Therefore, intrinsic viscosity is used herein to define the number average molecular weight range of the polymers which are effective to provide the hair spray compositions with their hair fixative properties when used at high concentrations.

The use of low molecular weight polymers reduces the viscosity of the hair spray composition and enhances the spray characteristics of hair spray compositions containing these polymers. Such reduction in viscosity typically results in reduced fixative properties, such as stiffness, i.e. initial curl droop, and humidity resistance. Additionally, the presence of significant levels of water in the hair spray compositions contributes to the reduction of these properties. The inventors have surprisingly discovered that by selecting certain molecular weight polymers of certain composition and using them at certain concentration ranges which are relatively higher than those ranges disclosed in the '397 and '199 patents, a synergistic result of good spray characteristics, good hold properties, hydrolytic stability and good removability can be achieved in low VOC hair spray compositions. The high polymeric solids content compensates for any lower fixative properties generally associated with lower molecular weight hair care resins, and therefore, there is minimum initial curl droop and good holding power, even though the compositions contain significant amounts of water.

The preferred molecular weight and concentration of polymer used are dependent upon each other and are further dependent on the composition of the polymer itself. For instance, acrylic-based polymers used in the inventive hair sprays will have an intrinsic viscosity in ethyl alcohol of from 0.075 to 0.200, preferably from 0.100 to 0.175. By acrylate-based it is meant that the polymers comprise at least 70%, preferably at least 85%, by weight of monomers selected from the group consisting of acrylic acid, methacrylic acid, $C_1$-$C_{12}$ alkyl esters of acrylic or methacrylic acid, and $C_1$-$C_8$ alkyl-substituted acrylamides and methacrylamides, based on the total weight of monomers. Vinyl ester-based polymers which are used will have an intrinsic viscosity in acetone of from 0.100 to 0.300. By vinyl ester-based it is meant that the polymers comprise at least 70%, preferably at least 85%, by weight of a vinyl ester of $C_2$-$C_{12}$ carboxylic acids, based on the total weight of monomers. Once armed with the teachings of the present specification, one skilled in the art will be able to ascertain proper molecular weight and concentration for use in the hair spray compositions while maintaining hair fixative properties.

The hair spray compositions of this invention comprise 5-20%, preferably 7-20%, more preferably 7-15% of a hydrolytically stable, low molecular weight polymer in an aqueous solvent system in which the solvent comprises a mixture of 80% or less volatile organic compounds by weight of the total hair spray composition and the remainder of water. The low molecular weight polymers are water soluble with neutralization and comprise monomers containing one or more available carboxyl groups, present in an amount of 5%-35%, preferably 5%-25%, by weight of the polymer; one or more water insoluble comonomers present in an amount of 65%-95% by weight of the polymer; and optionally, one or more non-ionic, water soluble comonomers present in an amount up to 20%, preferably up to 10%, by weight of the polymer.

Suitable monomers that contain carboxyl groups are the $C_3$-$C_{12}$ mono- or dicarboxylic acids, such as, acrylic acid, methacrylic acid, crotonic acid, itaconic acid, maleic acid, fumaric acid, and the $C_1$-$C_8$ alkyl half esters of maleic and fumaric acids, such as methyl hydrogen maleate and butyl hydrogen fumarate, and any other acidic monomers that are capable of being copolymerized with the particular desired interpolymer system. The preferred carboxyl containing monomers are acrylic acid, crotonic acid, and monoisopropyl maleate.

The water insoluble comonomers suitable for use with the carboxyl containing monomers are $C_1$-$C_{12}$ alkyl esters of acrylic acid and methacrylic acid; $C_1$-$C_8$ alkyl substituted acrylamides and methacrylamides; vinyl esters of $C_2$-$C_{12}$ carboxylic acids, styrene, and combinations of them. The preferred water insoluble comonomers are vinyl acetate, vinyl pivalate, vinyl versatate, vinyl neodecanoate, methyl methacrylate and t-octyl acrylamide.

The nonionic water soluble comonomers suitable for use in the polymers are water soluble $C_2$-$C_4$ hydroxyalkyl esters of acrylic and methacrylic acids, $C_1$-$C_4$ alkyl $C_2$-$C_4$ aminoalkyl esters of acrylic and methacrylic acids, acrylamide, dimethyl acrylamide, N-vinyl pyrrolidone and vinyl caprolactam. The preferred water soluble comonomers are hydroxypropyl methacrylate and hydroxyethyl methacrylate.

The low molecular weight polymers suitable for this application are prepared by solution polymerization with uniform slow addition of monomers and initiators, employing a higher concentration of short half-life ($t\frac{1}{2}$) initiator than would be used for the preparation of high molecular weight polymers. Exemplary initiators are t-amyl peroxypivalate (a product of Atochem Inc.) and 2,2'-azobis(2-methylpropanenitrile), sold under the tradename Vazo 64 by duPont. Typical one hour half-life ($t\frac{1}{2}$) temperature of the initiators are in the range of 60°C to 85°C, half-life being defined as the time it takes for one half of a given quantity of initiator to decompose.

The hair spray compositions are prepared by neutralizing the polymer in water with one or more cosmetically acceptable organic or inorganic bases known and used in the art. The amount of base will be an effective amount to effect subsequent removability from the hair and a determination of the exact amount is within the expertise of one skilled in the art. The preferred bases are sodium hydroxide, potassium hydroxide, 2-amino-2-methyl-1-propanol, histidine, tris(hydroxymethyl)aminomethane dimethyl stearamine, 2-amino-2-methyl-1,3-propanediol and triethanolamine.

The hair spray compositions are designed to be aqueous systems containing 80% or less volatile organic compounds. The amount and choice of organic solvent used will depend on the desired end product formulation. A propellant may also be used when it is desired to deliver the hair spray formulation in an aerosol delivery system. In some instances, the propellant may also function as a solvent. Suitable solvents are one or more alcohols, ethers and ketones, such as, ethanol, isopropanol, dimethoxymethane, acetone, and methyl ethyl ketone. Suitable propellants are ethers, such as dimethyl ether, compressed gases, halogenated hydrocarbons and hydrocarbons.

The invention is best described through the following examples. The examples illustrate the syntheses of representative polymers, and their performance in hair spray compositions in comparison to the performance of higher molecular weight polymers in the same hair spray compositions. The viscosities of the polymers were measured on a Brookfield viscometer. The test protocol for measuring humidity resistance is given below.

**Test Protocol for Initial Curl Droop and Humidity Resistance.**

The strength and humidity resistance of the low molecular weight polymers in hair spray formulations at various solids levels in 80% and 55% VOC systems was determined by measuring initial curl droop in the first six minutes after spraying and curl retention in a high humidity cabinet over a 24 hour period. The procedure for preparing the curls was as follows: Each of the hair spray systems was tested on nine swatches of six inch strands of Remi Blue String European Brown hair approximately 2 grams in weight and the results pooled and averaged. The hair was washed in a 10% solution of shampoo, rinsed and dried at 49°C (120°F). It was wet again, combed, and rolled and secured onto a 1/2 inch diameter Teflon® mandrel. It was dried at 49°C (120°F) and removed from the mandrel.

To measure initial curl droop for each swatch, each curl was suspended, the curl height was measured and then sprayed for uniform polymer deposition. The curl droop was measured as percent curl retention at 30, 60, and 90 second intervals, and at 2,4, and 6 minute intervals.

To measure humidity resistance, the curl was sprayed uniformly with four bursts in the case of non-aerosol embodiments and 2 seconds per side in the case of aerosol embodiments. The curl was laid on a horizontal surface and allowed to air dry for one hour. The dried curl was suspended and set into a chamber at 70°F, 90% relative humidity, and the curt height measured immediately, and at 15, 30, 60 and 90 minute intervals, and 2, 3, 4, 5 and 24 hour intervals.

The percentage curl retention was calculated by the formula $(L-L^t)/(L-L°) \times 100$, where L is the length of hair fully extended, $L°$ is the length of hair before spray and exposure, and $L^t$ is the length of hair after spray and exposure.

**Test Protocol for Shampoo Removability**

Eight (8) 10 inch Remi Blue String European Brown hair swatches weighing approximately 3 grams each were provided per test sample. From a distance of about 6 inches, each swatch was sprayed for a 2-second burst per side in the case of aerosol embodiments and 10 bursts per side in the case of non-aerosol embodiments. The swatches were allowed to dry for approximately one hour. Each swatch was wet with warm tap water, with the excess water being removed from the swatch. Three drops of a shampoo formulation containing 40 weight percent sodium laureth sulfate, 27.6 weight percent sodium lauryl sulfate, 0.5 weight percent cocoamide DEA, 0.1 weight percent methylchloroisothiazolinone and methylisothiazolinone and 31.8 weight percent deionized water were applied along the length of each swatch and worked into a lather for 30 seconds. Each swatch was rinsed with water for 30 seconds. Excess water was removed from the swatches and each swatch was placed in an oven at 110°F until dry. Each swatch was then evaluated for stiffness and flake.

**Test Protocol for Intrinsic Viscosity**

Acrylic-based polymers:

To a 125 ml Erlenmeyer flask were added 0.8± 0.1 grams of the acrylic-based polymer. To the flask were added 100 ml of filtered reagent grade SDA-4-2 anhydrous ethanol and the flask was swirled until a water clear solution was obtained. Five (5) ml of the solution were transferred to a Cannon 100 H 990 Viscometer having a bath temperature of 30°C± 0.2°C. The temperature of the solution was allowed to equilibrate for about 25 minutes. The flow time of the solution was then determined. Polymer solution concentration was determined using three 10 ml aliquots.

Vinyl-ester-based polymers:

The above procedure was followed with the following exceptions. The SDA-40-2 anhydrous ethanol was replaced with acetone. Approximately one (1) gram of polymer was used per test.

Intrinsic viscosity was calculated as below:

$$I.V.= \frac{3[(\eta \text{ rel.})^{1/3}-1]}{C}$$

$$\eta \text{ rel.} = \frac{\text{Flow time of solution in seconds}}{\text{Flow time of solvent in seconds}}$$

$$C = \text{concentration of solution (g/ml)}$$

**Example I.**

**Acrylic Polymer Compositions and Preparation**. A series of acrylate polymers was made with the monomer compositions disclosed in Table I according to the following procedure: A reaction vessel equipped with a thermometer, mechanical mixer and condenser was charged with 11 parts of the monomer mix, 27 parts ethanol and 0.5 parts of 75% tertiary-amyl peroxypivalate in odorless mineral spirits. The reactor contents were heated to the reflux temperature of the system (78°C). The remainder of the monomer mix was slow added uniformly over six (6) hours along with a second slow add stream consisting of 5 parts of 75% tertiary-amyl peroxypivalate (in odorless mineral spirits and 10 parts ethanol). When all slow adds were complete, the reaction was held at reflux temperature for two (2) hours. Then, 33 parts of isopropyl acetate and 7.5 parts of ethanol were added. The polymer was recovered by precipitating into water and drying at 60°C overnight.

5

Table I

| Acrylic Polymers | | | | | | |
|---|---|---|---|---|---|---|
| Monomers in parts by weight | | | | | | |
| Polymer | tOA | MMA | AA | HPMA | tBAEM | IV |
| A | 50 | 20 | 15 | 10 | 5 | .12 |
| B | 45 | 20 | 20 | 10 | 5 | .11 |
| C | 50 | 20 | 20 | 5 | 5 | .12 |
| D | 45 | 25 | 15 | 10 | 5 | .12 |
| E | 50 | 25 | 20 | 0 | 5 | .11 |
| F | 50 | 25 | 15 | 5 | 5 | .11 |
| G | 40 | 25 | 20 | 10 | 5 | .16 |
| H | 50 | 25 | 15 | 5 | 5 | .12 |
| I | A high molecular weight commercial tOA/acrylate/tBAEM copolymer | | | | | .40 |

Notes to Table I: The intrinsic viscosity (IV) was measured in ethanol. tOA is tertiary octylacrylamide; MMA is methyl methacrylate; AA is acrylic acid; HPMA is hydroxypropyl methacrylate; and tBAEM is tertiary butylaminoethyl methacrylate.

**Spray Characteristics/Particle Size Analysis.** It is known to those skilled in the art that a smaller particle size is known to translate to better spray characteristics for hair fixative polymers. Acrylate Polymer E was formulated into a series of aerosol and nonaerosol hair sprays, sprayed from either an aerosol actuator or nonaerosol pump and analyzed for particle size on a Malverne Master Sizer (from Malverne Instruments Inc., Southborough, MA). The results were compared to a commercial high molecular weight acrylate polymer. The hair spray formulations and the results of the comparisons are set out in Table II. The data show that for every direct comparative example Polymer E had a smaller median particle size than Polymer I, and that at higher solids concentration utilizing ethanol, n-butane and dimethylether as the delivery system, the commercial polymer was too viscous to obtain spray.

Table II

| Formulation | Parts by weight | | | | | | |
|---|---|---|---|---|---|---|---|
| **Spray Characteristics/Particle Size** | | | | | | | |
| Polymer I | 4.00 | | | 8.00 | 8.00 | | |
| Polymer E | | 4.00 | 4.00 | | | 8.00 | 8.00 |
| 2-Amino-2-methyl-- 1- propanol | .79 | .71 | .71 | 1.57 | 1.57 | 1.43 | 1.43 |
| Water | 15.21 | 15.29 | 15.29 | 10.43 | 10.43 | 10.57 | 10.57 |
| Ethanol (anhydrous) | 80.00 | 50.00 | 80.00 | 50.00 | 80.00 | 50.00 | 80.00 |
| n-Butane | | 12.00 | | 12.00 | | 12.00 | |
| Dimethylether | | 18.00 | | 18.00 | | 18.00 | |
| Valve | A2 | A1 | A2 | A1 | A2 | A1 | A2 |
| Median Particle Size (microns) | 65.6 | 45.3 | 54.2 | ** | 88.2 | 52.1 | 73.5 |

Notes to Table 2. **Too viscous to spray. The hair spray formulations are given in parts by weight. Valve A1 is an aerosol Precision valve with the following dimensions: 0.016" stem; 0.016" body; 8" dip tube; Kosmos actuator 0.016" orifice MB concave delta. Valve A2 is a Seaquist pump, EuroMist II actuator (190 mcl output), 0.018" x 0.010" shallow orifice dimensions.

EP 0 638 306 A1

**Humidity Resistance.** The humidity resistance of low molecular weight acrylate polymers disclosed in Table I was tested by measuring curl retention in an 80% VOC system consisting of 18% dimethyl ether, 50% ethanol, 12% n-butane at 5% solids. The percentage curl retentions are given in Table III and show acceptable performance in humid environments.

Table III

| Humidity Resistance | | | | | | | | | |
|---|---|---|---|---|---|---|---|---|---|
| Percentage Curl Retention | | | | | | | | | |
| Polymer | 15 min | 30 min | 60 min | 90 min | 2 hr | 3 hr | 4 hr | 5 hr | 24 hr |
| H | 90.45 | 88.55 | 86.58 | 86.03 | 85.41 | 84.79 | 84.14 | 83.58 | 82.97 |
| E | 94.95 | 93.75 | 93.10 | 92.44 | 91.82 | 91.21 | 91.21 | 91.21 | 91.21 |
| D | 90.54 | 88.75 | 86.24 | 86.24 | 86.24 | 86.24 | 86.24 | 85.62 | 85.00 |
| G | 93.17 | 91.32 | 88.19 | 88.19 | 86.34 | 85.72 | 85.10 | 85.10 | 83.87 |
| B | 91.35 | 88.91 | 85.74 | 83.34 | 82.72 | 81.49 | 80.87 | 80.87 | 78.98 |
| C | 93.72 | 90.59 | 87.47 | 87.47 | 86.88 | 84.96 | 83.69 | 83.69 | 83.07 |

**Example II**

**Vinyl Ester Polymer Compositions and Preparation.** A series of vinyl acetate polymers was made with the monomer compositions disclosed in Table IV according to the following procedure: A reaction vessel equipped with a thermometer, mechanical mixer and condenser was charged with 49.2 parts of monomer mix, 15.4 parts of ethyl acetate and 0.76 parts of t-butyl peroxy pivalate. The contents were heated to the reflux temperature, and the remaining monomer mix slow added uniformly over four hours along with a second slow add stream consisting of a mixture of 0.84 parts t-butyl peroxy pivalate and 14 parts ethyl acetate. When slow adds were complete, the reaction was held at reflux temperature for one hour. A mixture of 0.3 parts t-butyl peroxy pivalate and 5 parts ethyl acetate was added to the reactor and the contents heated for six hours. The contents were cooled to 60°C while adding 16 parts ethyl acetate. The polymer was recovered by precipitating into water and drying at 60°C overnight.

Table IV

| Vinyl Ester Polymers | | | | | | |
|---|---|---|---|---|---|---|
| Monomers in parts by weight | | | | | | |
| Polymer | VA | CA | VV-10 | VV-9 | VP | IV |
| 1 | 76.5 | 10.0 | 0 | 13.5 | 0 | 0.309 |
| 2 | 48.0 | 15.0 | 0 | 0 | 37.0 | 0.263 |
| 3 | 30.0 | 10.0 | 30.0 | 0 | 30.0 | 0.194 |
| 4 | 48.0 | 15.0 | 0 | 0 | 37.0 | 0.303 |
| 5 | 76.5 | 10.0 | 0 | 0 | 13.5 | 0.340 |
| 6 | 69.0 | 10.0 | 0 | 0 | 22.0 | 0.474 |
| 7 | 38.0 | 8.0 | 0 | 0 | 44.0 | 0.236 |
| 8 | 30.0 | 10.0 | 60.0 | 0 | 0 | 0.199 |
| 9 | 41.0 | 15.0 | 0 | 0 | 44.0 | 0.150 |
| 10 | 61.0 | 10.0 | 0 | 0 | 30.0 | 0.339 |
| 11 | 30.0 | 10.0 | 30.0 | 30.0 | 0 | 0.149 |
| 12 | 35.0 | 15.0 | 0 | 0 | 50.0 | 0.270 |
| 13 | 42.0 | 10.0 | 0 | 48.0 | 0 | 0.224 |
| 14 | 30.0 | 10.0 | 0 | 60.0 | 0 | 0.143 |
| 15 | 35.0 | 15.0 | 0 | 0 | 50.0 | 0.110 |
| 16 | 30.0 | 10.0 | 0 | 30.0 | 30.0 | 0.178 |
| 17 | 36.0 | 10.0 | 0 | 54.0 | 0 | 0.191 |
| 18 | Commercially available vinyl acetate/crotonic acid/vinyl neodecanoate copolymer | | | | | 0.311 |
| Notes to Table IV. VA is vinyl acetate; CA is crotonic acid; VV-9 is neononanoic acid; VV-10 is vinyl neodecanoate; VP is vinyl pivalate; IV is intrinsic viscosity in acetone. | | | | | | |

**Spray Characteristics/Solution Viscosity.** It is known to those skilled in the art that solution viscosity of the hair fixative composition affects spray characteristics, and that the lower the viscosity the better the spray. The viscosity of Polymer 9 in water at varying solids levels was compared to the viscosity of commercial Polymer 18 in water. The results of the comparison are set out in Table V and show that the viscosity of low molecular weight Polymer 9 at 15% solids is comparable to that of high molecular weight commercial Polymer 18 at 5% solids. Thus, the data show that it would be possible to put up to three times as much of the low molecular weight Polymer 9 into the hair fixative composition, compared to Polymer 18, and still maintain similar spray characteristics. This would allow one to reduce VOC levels without sacrificing properties of stiffness or humidity resistance. On the other hand, the data also shows that it would not be possible to include higher concentrations of Polymer 18 in the hair fixative compositions and still maintain functional spray characteristics.

Table V

| Solution Viscosity | | |
|---|---|---|
| Solids Level | Polymer 9 | Polymer 18 |
| 5% | 4.7 mPa.s | 10.1 mPa.s |
| 8% | 6.5 mPa.s | 16.8 mPa.s |
| 10% | 7.3 mPa.s | 20.6 mPa.s |
| 12% | 9.0 mPa.s | 30.5 mPa.s |
| 15% | 12.7 mPa.s | 43.5 mPa.s |

**Initial Curl Droop/Humidity Resistance Relative to Solids Levels.** The stiffness and humidity resistance of a low molecular weight vinyl acetate Polymer 9 disclosed in Table IV was tested relative to varying solids levels by measuring percentage curl retention as described below. The polymer was neutralized 100% with 2-amino-2-methyl-1-propanol (AMP) and formulated into 80% and 55% VOC systems. The 80% VOC system comprised 18% dimethyl ether, 50% ethanol, and 12% n-butane. The 55% VOC system comprised 22% ethanol and 33% dimethyl ether.

In the initial curl droop test (a measure of stiffness), Polymer 9 at varying solids levels was compared to commercial Polymer 18 at 5% solids in an 80% VOC system and in an anhydrous system. The percentage curl retention (initial curl droop) are set out in Table VI. The results show that in 80% VOC formulations, the amount of initial curl droop decreased with increasing resin concentration. The hair spray formulation containing Polymer 9 at 11% solids was statistically equivalent to the anhydrous control, and the hair spray formulations at 13% and 15% solids Polymer 9 were superior to the anhydrous control. In 55% VOC systems, the amount of initial curl droop was also reduced as the polymer solids level was raised. These effective levels of polymer solids would not be possible in typical high molecular weight polymer systems because of the high viscosity of the high molecular weight systems.

## Table VI: Initial Curl Droop

| Sample | Percent Curl Retention | | | | | |
|---|---|---|---|---|---|---|
| | 30 sec | 60 sec | 90 sec | 2 min | 4 min | 6 min |
| % solids<br>% VOC | | | | | | |
| 15% Polymer 9<br>80% VOC | 97.4 | 97.4 | 97.4 | 96.5 | 96.5 | 96.5 |
| 13% Polymer 9<br>80% VOC | 95.6 | 95.6 | 95.6 | 95.6 | 95.6 | 93.9 |
| 11% Polymer 9<br>80% VOC | 94.8 | 94.0 | 94.0 | 93.1 | 90.6 | 87.9 |
| 9% Polymer 9<br>80% VOC | 95.8 | 94.1 | 93.2 | 93.2 | 89.1 | 85.6 |
| 7% Polymer 9<br>80% VOC | 95.0 | 94.2 | 93.3 | 93.3 | 87.5 | 84.2 |
| 5% Polymer 9<br>80% VOC | 93.2 | 93.2 | 92.5 | 92.5 | 83.5 | 76.2 |
| 5% Polymer 18<br>80% VOC | 93.4 | 92.7 | 92.7 | 91.9 | 88.3 | 80.3 |
| | | | | | | |
| 5% Polymer 18<br>anhydrous | 97.0 | 96.2 | 96.2 | 96.2 | 93.3 | 92.6 |
| | | | | | | |
| 15% Polymer 9<br>55% VOC | 97.4 | 94.7 | 92.2 | 92.2 | 89.5 | 87.7 |
| 13% Polymer 9<br>55% VOC | 95.9 | 92.6 | 91.7 | 90.9 | 90.7 | 89.3 |
| 11% Polymer 9<br>55% VOC | 93.0 | 90.4 | 88.7 | 86.0 | 83.4 | 83.4 |
| 9% Polymer 9<br>55% VOC | 95.0 | 92.1 | 86.9 | 85.5 | 83.4 | 83.4 |
| 7% Polymer 9<br>55% VOC | 92.1 | 85.7 | 80.5 | 76.1 | 69.9 | 69.0 |
| 5% Polymer 9<br>55% VOC | 90.2 | 83.9 | 77.6 | 70.3 | 59.6 | 58.7 |
| | | | | | | |
| 5% Polymer 18<br>80% VOC | 94.2 | 94.2 | 94.2 | 94.2 | 86.9 | 81.6 |
| | | | | | | |
| 5% Polymer 18<br>anhydrous | 95.3 | 95.3 | 95.3 | 95.3 | 95.3 | 92.9 |

The humidity resistance (percentage curl retention) of Polymer 9 at varying solids levels was compared to Polymer 18 at 5% solids in an 80% VOC system and in an anhydrous system. The percentage curl retentions are set out in Table VII. The results show that at all solids concentrations Polymer 9 in the 80% VOC systems performed significantly superior (at the 95% confidence level) to Polymer 18. Hair spray samples containing Polymer 9 at 7% and higher solids were significantly superior to the anhydrous Polymer 18 system at all time intervals. After 90 minutes in the humidity chamber, the Polymer 9 system at 5% solids became significantly superior to the anhydrous Polymer 18 system.

**Table VII: Humidity Resistance**

| % solids % VOC | Percent Curl Retention | | | | | | | | |
|---|---|---|---|---|---|---|---|---|---|
| | 15 min | 30 min | 60 min | 90 min | 2 hr | 3 hr | 4 hr | 5 hr | 24 hr |
| 10%Polymer9 80% VOC | 96.9 | 93.8 | 89.8 | 89.0 | 87.5 | 86.6 | 85.8 | 85.0 | 81.8 |
| 9% Polymer9 80% VOC | 96.8 | 95.3 | 89.7 | 88.9 | 88.2 | 87.4 | 86.6 | 86.6 | 84.2 |
| 8% Polymer9 80% VOC | 95.3 | 92.1 | 89.7 | 86.5 | 85.7 | 84.9 | 84.9 | 84.9 | 84.1 |
| 7% Polymer9 80% VOC | 96.0 | 92.1 | 89.7 | 87.4 | 86.6 | 86.6 | 85.8 | 84.9 | 82.6 |
| 6% Polymer9 80% VOC | 92.1 | 89.0 | 85.9 | 83.6 | 82.1 | 81.3 | 81.3 | 81.3 | 79.8 |
| 5% Polymer9 80% VOC | 90.6 | 86.1 | 82.6 | 82.6 | 81.1 | 78.2 | 78.2 | 77.5 | 77.5 |
| 5% Polymer 18 80% VOC | 84.9 | 79.9 | 72.0 | 70.6 | 68.4 | 65.7 | 64.9 | 64.9 | 52.1 |
| 5% Polymer 18 Anhydrous | 88.6 | 83.0 | 78.1 | 75.2 | 73.0 | 69.5 | 68.0 | 68.0 | 61.1 |

**Hydrolytic Stability.** A shortcoming of many of the current hair fixative resins containing vinyl acetate is their lack of stability in aqueous solution due to hydrolysis of the vinyl acetate. To test for hydrolytic stability, the pH of low molecular weight vinyl acetate polymers disclosed in Table IV was monitored over a 9 week period. The samples were formulated at 7.5% solids in water, neutralized 110% with AMP, and stored in a 43.5°C (110°F) oven. The results are set out in Table VIII and show that these polymers retained hydrolytic stability for the term of the test.

Table VIII

| Hydrolytic Stability | | | | | | |
|---|---|---|---|---|---|---|
| Polymer | IV* | pH Initial | 2 weeks | 4 weeks | 9 weeks | pH Drop |
| 3 | .194 | 8.6 | 8.8 | 8.6 | 8.5 | 0.1 |
| 11 | .149 | 9.2 | 8.9 | 8.6 | 8.6 | 0.6 |
| 16 | .178 | 8.5 | 8.7 | 8.5 | 8.5 | 0 |
| 8 | .199 | 8.8 | 8.6 | 8.3 | 8.2 | 0.6 |
| 14 | .143 | 8.7 | 8.8 | 8.6 | 8.6 | 0.1 |

* IV is intrinsic viscosity.

**Subjective Properties.** An 80% VOC hair spray formulation containing 10% Polymer 9 was compared to 80% VOC hair spray formulations containing 5% Polymer 9 and 5% Polymer 18 for each of the characteristics of stiffness, resistance to combing, accumulation of flake, gloss and static.

The samples were evaluated on 10 inch long, 2 gram swatches of brown hair by a panel of 8 persons. Each panel member evaluated a test swatch treated with one of the example formulations and a control swatch treated with a control formulation. The panel member rated both the test swatch and the control swatch by a numerical performance rating, and equivalent ratings were not permitted. In the evaluations, stiffness was superior to softness; no resistance to combing was superior to resistance; no flake accumulation on hair and comb was superior to flake accumulation; gloss was superior to lack of gloss; and no static flyaway after combing was superior to static flyaway. A total of 8 repeats per sample were made. The panel results were analyzed statistically (95% confidence level) and summarized comparing the test swatch as superior to (+), equivalent to (=), or inferior to (-), the control swatch. The results of the panel evaluations are set out in Table IX and show that the hair spray formulation with the low molecular weight polymer performed better or comparably to the formulation with the higher molecular weight polymer and comparably to the lower solids formulation with the lower molecular weight polymer.

Table IX

| Subjective Evaluation in 80% VOC Systems | | | | | |
|---|---|---|---|---|---|
| 10% Polymer 9 | Gloss | Stiffness | Dry Comb | Flake | Antistatic |
| vs. 5% Polymer 9 | 7/8 + | 7/8 + | 1/8 - | 3/8 = | 3/8 = |
| vs. 5% Polymer 18 | 4/8 = | 3/8 = | 4/8 = | 0/8 = | 7/8 + |

It should be recognized that changes and modifications in the specifically described embodiments can be carried out without departing form the scope of the invention, which is intended to be limited only by the scope of the appended claims.

**Claims**

1. A hair spray composition, comprising:
   (a) 7-20% by weight, based on the total weight of the hair spray composition, of a hydrolytically stable, hydrophobic, low molecular weight, acrylic-based or vinyl ester-based polymer having an intrinsic viscosity of 0.100-0.300; and
   (b) a solvent system comprising 80% or less of a volatile organic compound by weight of the hair spray composition and the remainder of water.

2. The hair spray composition of Claim 1 further comprising an effective amount of a cosmetically acceptable base to effect subsequent removability of the polymer from hair to which the hair spray composition has been applied.

3.  The hair spray composition of Claim 2 wherein the polymer further comprises residues of one or more non-ionic, water soluble comonomer(s) selected from the group consisting of $C_2$-$C_4$ hydroxyalkyl esters of acrylic and methacrylic acids, $C_1$–$C_4$ alkyl $C_2$-$C_4$ aminoalkyl esters of acrylic and methacrylic acids, acrylamide and methacrylamide, dimethyl acrylamide and methacrylamide, N-vinyl pyrrolidone and vinyl caprolactam.

4.  The hair spray composition of Claim 2 further comprising a propellant selected from the group consisting of ethers, compressed gases, halogenated hydrocarbons and hydrocarbons.

5.  The hair spray composition of Claim 1 wherein the polymer comprises the reaction product of
    (a) one or more acidic monomers selected from the group consisting of $C_3$-$C_{12}$ mono-and dicarboxylic acids and the $C_1$-$C_8$ alkyl half esters of maleic and fumaric acids, present in an amount of 5% - 35% by weight of the polymer; and
    (b) one or more water insoluble comonomer(s) selected from the group consisting of $C_1$-$C_{12}$ alkyl esters of acrylic acid and methacrylic acid, $C_1$-$C_8$ alkyl substituted acrylamides and methacrylamides, vinyl esters of $C_3$-$C_{12}$ carboxylic acids, and styrene, present in an amount of 65% - 95% by weight of the polymer; and optionally
    (c) up to 20% by weight of polymerized residues of one or more water soluble comonomers selected from the group consisting of water soluble hydroxyalkyl esters of acrylic and methacrylic acids, $C_1$-$C_4$ alkyl, $C_2$-$C_4$ aminoalkyl esters of acrylic and methacrylic acids, acrylamide and methacrylamide, dimethyl acrylamide and methacrylamide, N-vinyl pyrrolidone, and vinyl caprolactam.

6.  The hair spray composition of Claim 5 further comprising an effective amount of a cosmetically acceptable base to effect subsequent removability of the polymer from hair to which the hair spray composition has been applied.

7.  The hair spray composition of Claim 6 wherein the cosmetically acceptable base for neutralization is selected from the group consisting of sodium hydroxide, potassium hydroxide, 2-amino-2-methyl-1-propanol, tris(hydroxymethyl)aminomethane, histidine, dimethyl stearamine, 2-amino-2-methyl-1,3-propanediol, and triethanolamine.

8.  The hair spray composition of Claim 6 further comprising a propellant selected from the group consisting of ethers, compressed gases, halogenated hydrocarbons and hydrocarbons.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO-A-92 13014 (ISP INVESTMENTS) 6 August 1992 --- | 1 | A61K7/06 |
| X | WO-A-93 06816 (ISP INVESTMENTS) 15 April 1993 --- | 1,3,4 | |
| P,X | WO-A-94 02112 (NATIONAL STARCH) 3 February 1994 --- | 1-8 | |
| X | US-A-5 182 098 (KOPOLOW ET AL.) 26 January 1993 --- | 1 | |
| A | EP-A-0 135 983 (GAF CORP.) 3 April 1985 ----- | | |

**TECHNICAL FIELDS SEARCHED** (Int.Cl.6)

A61K

The present search report has been drawn up for all claims

| Place of search | Date of completion of the search | Examiner |
|---|---|---|
| THE HAGUE | 14 November 1994 | Klaver, T |

EPO FORM 1503 03.82 (P04C01)